Europäisches Patentamt

European Patent Office

Office européen des brevets

(19)

(11) EP 0 835 651 A2

(12) # DEMANDE DE BREVET EUROPEEN

(43) Date de publication:
15.04.1998 Bulletin 1998/16

(51) Int Cl.6: **A61K 7/48**, A61K 7/00

(21) Numéro de dépôt: 97402234.5

(22) Date de dépôt: 25.09.1997

(84) Etats contractants désignés:
AT BE CH DE DK ES FI FR GB GR IE IT LI LU MC NL PT SE

(30) Priorité: 11.10.1996 FR 9612450

(71) Demandeur: L'OREAL
75008 Paris (FR)

(72) Inventeurs:
• Sebillotte-Arnaud, Laurence
94240 L'Hay Les Roses (FR)

• Gagnebien, Didier
Westfield 07090, New Jersey (US)

(74) Mandataire: Tezier Herman, Béatrice
L'OREAL,
Département Propriété Industrielle,
90, rue du Gal Roguet
92583 Clichy Cédex (FR)

(54) **Emulsion cosmétique H/E à forte teneur en électrolytes**

(57) La présente invention se rapporte à une émulsion huile dans eau stable, à forte teneur en électrolytes, comprenant au moins 2 % en poids par rapport au poids total de la composition d'un sel métallique hydrosoluble, utile notamment pour une application topique, en particulier pour une utilisation en dermo-cosmétique, pour traiter les phénomènes d'irritation et/ou de peaux sensibles.

EP 0 835 651 A2

## Description

La présente invention se rapporte à une émulsion huile dans eau stable, à forte teneur en électrolytes, comprenant au moins 2 % en poids par rapport au poids total de la composition d'un sel métallique hydrosoluble, et un système émulsionnant approprié en quantité suffisante pour obtenir une composition stable, utile notamment pour une application topique. L'invention concerne également l'utilisation de la composition selon l'invention en dermocosmétique, notamment pour les désordres pathologiques et/ou physiologiques associés à la libération de la substance P et/ou du TNF-alpha (Tumor Necrosis Factor-alpha) et notamment de traiter les peaux sensibles, les désordres et maladies cutanées où existe un prurit, la rosacée et/ou l'érythème pudique.

Il est connu que certaines peaux sont plus sensibles que d'autres. Les symptômes des peaux sensibles étaient jusqu'à présent mal caractérisés et le problème de ces peaux était, de ce fait, mal défini, personne ne connaissant exactement le processus mis en cause dans la sensibilité -hyperréactivité cutanée non allergique- de la peau. Certains pensaient qu'une peau sensible était une peau qui réagissait aux produits cosmétiques et/ou dermatologiques, d'autres qu'il s'agissait d'une peau qui réagissait à plusieurs facteurs extérieurs, pas forcément liés aux produits cosmétiques et/ou dermatologiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, <u>36</u>, pages 45-49; et l'article de T. Agner et J. Serup, Clinical and Experimental Dermatology, 1989, <u>14</u>, pages 214-217. Mais ces tests ne permettaient pas de caractériser les peaux sensibles, que l'on assimilait à des peaux allergiques.

Les symptômes liés aux peaux sensibles ont été mis en évidence et décrits dans la demande de brevet FR 95 04268 déposée le 10 avril 1995 au nom de L'OREAL. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Il a été montré en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

Les peaux sensibles pouvaient être scindées en deux grandes formes cliniques, les peaux irritables et les peaux intolérantes. Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine. En général, ces signes sont associés à une peau sèche avec ou sans dartres, ou à une peau qui présente un érythème. Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyperséborrhéique ou acnéique, voire même rosacéiforme, avec ou sans dartres.

D'une manière générale, les peaux sensibles se définissent par une réactivité particulière de la peau. Cette hyperréactivité peut être notamment mise en jeu par des facteurs environnementaux, émotionnels, alimentaires ou encore par l'application ou le contact de produits cosmétiques ou dermatologiques. Cet état d'hyper-réactivité qui définit les peaux sensibles différencie celles-ci de la réactivité ubiquitaire provoquée par des agents irritants qui induisent une irritation de la peau chez la quasi totalité des individus.

Cet état d'hyper-réactivité est ressentie et reconnue par les individus qui en sont atteints comme une "peau sensible".

Les cuirs chevelus "sensibles" ont une sémiologie clinique plus univoque : les sensations de prurit et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Le prurit est un symptôme fréquent des dermatoses, occasionnant une gêne souvent notable pour le patient. lorsque le prurit est très sévère, la gêne peut être telle que le patient ne peut poursuivre son activité habituelle. En outre, le prurit peut être source de complications : excoriations qui peuvent se surinfecter, lichénification des zones prurigineuses, qui a pour conséquence d'installer la maladie dans un véritable cercle vicieux. Parmi les dermatoses fréquemment associées à un prurit, on peut citer l'eczéma, la dermatite atopique, les dermites de contact, les lichens plans, les prurigos, les urticaires, les toxidermies prurigineuses, certaines formes cliniques de psoriasis.

Le prurit est parfois le signe pathologique cutané prédominant comme dans les cas du prurit aquagénique, du prurit du cuir chevelu lors des états pelliculaires *(pityriasis capitis),* du prurit des hémodialysés, des insuffisants rénaux, des sidéens et des personnes atteintes d'obstructions biliaires, ou encore du prurit des manifestations paranéoplastiques de certains cancers.

De plus, le prurit est un signe fréquemment rencontré au cours de certaines affections parasitaires cutanées ou générales. Il peut s'agir par exemple de scabiose, de filariose, d'oxyurose ou encore de démodéciose cutanée.

Du fait que l'on connaissait mal les caractéristiques des peaux sensibles, il était jusqu'à présent très difficile de les traiter, et on les traitait indirectement, par exemple en limitant dans les compositions cosmétiques ou dermatologiques l'emploi de produits à caractère irritant tels que les tensioactifs, les conservateurs ou les parfums ainsi que certains actifs.

Jusqu'à ce jour, les prurits étaient traités à l'aide de préparations émollientes, de corticoïdes locaux, de puvathérapie ou encore d'antihistaminiques. Les corticoïdes locaux sont certes très efficaces pour calmer les symptômes mais malheureusement, leur effet n'est pas immédiat. De plus, ils présentent des effets secondaires souvent très pénalisants comme des atrophies, et exposent à des risques d'infections mycosique et/ou bactérienne. La puvathérapie est l'irradiation locale de la peau malade avec des UVA, après absorption d'une substance photosensibilisante (psoralène). Cette technique présente les inconvénients graves d'un photovieillissement pouvant entraîner des cancers de la peau. De plus, ce traitement n'est pas ambulatoire, obligeant les malades à se rendre couramment dans un centre spécialisé pendant toute la durée du traitement, ce qui est très contraignant et limite leur activité professionnelle. Les émollients ont un effet anti-prurigineux très modeste et sont peu efficaces lorsque les prurits sont importants. Par ailleurs, les antihistaminiques n'ont pas une efficacité constante et nécessitent une prise orale.

Il subsiste donc le besoin d'un traitement de ces affections de la peau, ne présentant pas ces inconvénients.

L'utilisation d'au moins un sel métallique, en particulier d'un métal alcalino-terreux, permettant de traiter efficacement le prurit ou les problèmes de "peau sensible", tout en remédiant aux inconvénients mentionnés ci-dessus a été décrite décrits dans la demande de brevet FR 95 04268 déposée le 10 avril 1995 au nom de L'OREAL.

Des compositions gélifiées à forte teneur en électrolytes sont décrites dans les demandes de brevet FR 96 00742 et FR 96 03094 déposées le 27 janvier 1996 et le 12 mars 1996 au nom de L'OREAL.

La présente invention concerne une nouvelle composition, émulsion huile dans eau (H/E) comprenant une forte teneur en sels métalliques, permettant en particulier de traiter les problèmes de "peau sensible", notamment de prurit. La composition selon l'invention est de préférence une composition cosmétique ou dermatologique.

Dans le domaine cosmétique, il est courant d'utiliser des crèmes constituées d'une émulsion H/E comportant une phase huileuse dispersée dans une phase aqueuse. Ces émulsions présentent fréquemment des problèmes de stabilité, rendant leur fabrication difficile. Aussi, différents moyens ont été envisagés pour remédier à cet inconvénient. Un moyen d'y remédier consiste à augmenter fortement la teneur en émulsionnant de ces émulsions. Or, on sait que les émulsionnants utilisés en grande quantité peuvent se montrer irritants vis-à-vis de certains types de peau. On comprendra aisément que de telles compositions ne sont pas adaptées pour une application sur des peaux sensibles, compte-tenu des problèmes évoqués ci-dessus.

Différentes compositions, émulsions H/E à forte teneur en électrolyte sont décrites dans l'état de la technique. Ainsi, la demande de brevet EP 530 531 (Benckiser), décrit une composition cosmétique sous forme d'émulsion H/E ou E/H comprenant un système émulsionnant et au moins 2 % (préférentiellement au moins 5 %) d'un sel hydrosoluble de métaux alcalins ou alcalino-terreux. Le sel est de préférence un sel de magnésium qui agit comme conservateur. L'invention objet de cette demande, permet d'obtenir des compositions cosmétiques sans conservateurs. Pour les émulsions H/E, les compositions comprennent de 5 à 10 % en poids de système émulsionnant, généralement 8 % en poids.

Il est donc important de pouvoir disposer d'une composition stable, émulsion H/E, comportant une forte teneur en électrolyte et appropriée pour une application topique, en particulier pour traiter les problèmes de peaux sensibles et de prurit.

La présente invention concerne donc une nouvelle composition sous forme d'émulsion H/E, comprenant au moins 2 % en poids, de préférence au moins 3 % en poids, plus préférentiellement au moins 5 % en poids par rapport au poids total de la composition d'au moins un sel métallique hydrosoluble, et de 2 à 4,5% en poids, préférentiellement de 2 à 4% en poids par rapport au poids total de la composition d'un système émulsionnant approprié, à l'exception des compositions comprenant 1 % en poids d'un gélifiant constitué par l'association d'un polyacrylamide, d'isoparaffine en $C_{13}$-$C_{14}$ et de laureth-7 (CTFA) (commercialisé sous la dénomination Sepigel 305 par la société SEPPIC).

Par sel métallique, on entend selon la présente invention un sel d'un métal, c'est à dire d'un corps simple susceptible de libérer des cations simples (Dictionnaire de la Chimie et de ses Applications, DUVAL & DUVAL, 3ème Edition, 1978, Technique et Documentation).

Les sels métalliques hydrosolubles sont plus particulièrement choisis parmi les sels hydrosolubles de métaux alcalins, de métaux alcalino-terreux, de métaux de transition et de métaux des groupes 13 et 14 de la classification périodique des éléments.

Comme sels hydrosolubles de métaux alcalins utiles selon l'invention, on peut citer en particulier les sels de lithium, de sodium ou de potassium.

Comme sels hydrosolubles de métaux alcalino-terreux utiles selon l'invention, on peut citer en particulier les sels de béryllium, de magnésium, de calcium, de strontium et/ou de baryum.

Comme sels hydrosolubles de métaux de transition utiles selon l'invention, on peut citer en particulier les sels de lanthanides, et les sels de métaux de la quatrième période de la classification périodique des éléments, comme les sels de magnésium, de cobalt ou de zinc.

Comme sels hydrosolubles de métaux des groupes 13 et 14 de la classification périodique des éléments utiles selon l'invention, on peut citer les sels d'aluminium et d'étain.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.

De manière préférentielle, les sels métalliques hydrosolubles selon l'invention sont choisis parmi les sels de lithium, de strontium, de barium, d'yttrium, de néodyme, de gadolinium, de manganèse et de zinc, plus préférentiellement de strontium.

Ces sels peuvent être par exemple des carbonates, des bicarbonates, des sulfates, des glycérophosphates, des borates, des chlorures, des nitrates, des acétates, des hydroxydes, des persulfates ainsi que des sels d'$\alpha$-hydroxyacides ou des sels d'acides de fruits (citrate, tartrate, lactate, malate), ou encore des sels d'acides aminés (aspartate, arginate, glucocholate, fumarate) ou des sels d'acides gras (palmitate, oléate, caséinate, béhénate).

De préférence le sel est choisi parmi les nitrates ou les chlorures, en particulier le nitrate de lithium, de strontium, de barium, d'yttrium, de néodyme, de gadolinium, de manganèse ou de zinc, le chlorure de lithium, de strontium, de barium, d'yttrium, de néodyme, de gadolinium, de manganèse ou de zinc, les sulfates ou les acétates, comme le sulfate de calcium de strontium ou de magnésium et l'acétate de strontium ou de magnésium.

D'une manière avantageuse, la quantité de sels métalliques hydrosolubles dans la composition selon l'invention est comprise entre 2 % et 20 % en poids par rapport au poids total de la composition, de préférence comprise entre 5 % et 10 % en poids.

Par système émulsionnant approprié, on entend tout agent émulsionnant ou mélange d'agents émulsionnants susceptibles de produire une émulsion H/E à forte teneur en électrolytes stable lorsqu'ils sont présents dans la composition en une teneur comprise entre 2 et 4,5 % en poids par rapport au poids total de la composition.

Par émulsion H/E stable, on entend de préférence selon l'invention une émulsion qui reste stable au moins 1 mois à 45°C.

D'une manière avantageuse, le système émulsionnant approprié est constitué par au moins un ester d'acide gras et de polyéthylène glycol (PEG) et/ou au moins un ester d'acide gras et de polyol polyoxyéthyléné, appropriés pour la préparation d'émulsions H/E. Le système émulsionnant peut comprendre en outre au moins un ester d'acide gras et de polyol.

De manière préférentielle, le système émulsionant approprié est constitué par au moins un ester d'acide gras et de PEG et au moins un ester d'acide gras et de polyol.

Par acide gras, on entend de préférence selon l'invention les acides aliphatiques linéaires ou ramifiés, saturés ou insaturés, comprenant au moins 10 atomes de carbone, de préférence entre 10 et 20 atomes de carbone, plus préférentiellement entre 12 et 18 atomes de carbone. Il s'agit plus particulièrement des acides laurique, palmitique, stéarique, isostéarique et oléique.

Par ester d'acide gras et de PEG, on entend de préférence selon l'invention les esters d'acides gras tels que définis ci-dessus, et de PEG comprenant n unités PEG, n étant un entier au moins égal à 8, de préférence compris entre 10 et 100, plus préférentiellement compris entre 20 et 100. Des esters d'acides gras et de PEG préférés selon l'invention sont les stéarates de PEG-n, n étant un entier compris entre 8 et 100, commercialisés sous la dénomination Myrj par la société ICI, plus préférentiellement le stéarate de PEG-20 ou de PEG-40, commercialisés sous la dénomination Myrj 49 et Myrj52 par la société ICI.

Parmi les esters d'acide gras et de polyol polyoxyéthylénés (n-OE, n est un entier supérieur ou égal à 2 ; OE représente l'unité d'oxyéthylène), on peut notamment citer le tristéarate de sorbitane oxyéthyléné (20-OE), le trioléate de sorbitane oxyéthyléné (20-OE), le monolaurate de sorbitane oxyéthyléné (5-OE), le monostéarate de sorbitan oxyéthyléné (20-OE), le monopalmitate de sorbitane oxyéthyléné (20-OE) et le monooléate de sorbitane oxyéthyléné (20-OE).

Par ester d'acide gras et de polyol, on entend de préférence selon l'invention les mono ou polyesters d'acides gras tels que définis ci-dessus et de polyols.

Par polyols, on entend de préférence selon l'invention les alcools polyhydriques en $C_3$-$C_6$, comprenant 3 à 6 hydroxyles. Il s'agit plus particulièrement des sucres et du glycérol.

Les sucres sont avantageusement choisis parmi les sucres d'origine naturelle ou synthétique en $C_5$-$C_6$, plus particulièrement le sorbitol. De manière préférentielle, les esters d'acide gras et de sucre sont choisis parmi les mono- ou triesters d'acides gras et de sorbitol, en particulier ceux commercialisés sous les dénominations Arlacel ou Span par la société ICI. De manière particulièrement avantageuse, l'ester d'acide gras et de sucre est le tristéarate de sorbitol, en particulier commercialisé sous la dénomination Span 65 par la société ICI.

Parmi les esters d'acide gras et de glycérol, on peut citer les mono- ou diesters d'acide gras et de glycérol, en particulier le mono- ou distéarate de glycérol, et leurs mélanges en toutes proportions.

De préférence, le rapport pondéral ester d'acide gras et de PEG / ester d'acide gras et de polyol est compris entre 0,2 et 3, de préférence compris entre 0,25 et 2,5.

Selon un premier mode de réalisation de l'invention, le système émulsionant approprié est constitué par l'association de stéarate de PEG-40 et de tristéarate de sorbitol, le rapport pondéral stéarate de PEG / tristéarate de sorbitol étant compris entre 0,2 et 3, de préférence entre 0,25 et 2.

Selon un deuxième mode de réalisation de l'invention, le système émulsionant est constitué par l'association de stéarate de PEG-20 et d'un mélange de stéarate de PEG-100 et de mono/distéarate de glycérol (en particulier commercialisé sous la dénomination Arlacel 165 par la société ICI), le rapport pondéral stéarate de PEG-20/mélange étant compris entre 0,2 et 3, de préférence entre 0,25 et 1,5.

De préférence, le système émulsionnant présente une balance HLB supérieure ou égale à 9, avantageusement supérieure ou égale à 11. De préférence, le système émulsionnant présente une balance HLB inférieure ou égale à 18. On calcule la balance HLB (balance hydrophile-lipophile) d'un émulsionnant suivant la formule suivante :

$$HLB = \frac{100-L}{5}$$

dans laquelle L représente le pourcentage en poids du groupement lipophile par rapport au poids de la molécule entière. Lorsque plusieurs émulsionnants sont présents dans le système émulsionnant, la HLB du système émulsionant correspond à la HLB moyenne calculée en fonction de la quantité des émusionnants présents.

La phase aqueuse de l'émulsion selon l'invention peut comprendre de l'eau, de l'eau purifiée, une eau florale telle que l'eau de bleuet, ou une eau thermale ou minérale naturelle et leurs mélanges. Par exemple l'eau thermale ou minérale naturelle peut être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades, l'eau de Tercis-les-bains, l'eau d'Avène ou l'eau d'Aix les Bains.

La phase aqueuse est de préférence constituée par une eau thermale connue pour ses propriété apaisantes, anti-irritantes, anti-radicalaires, appliquée sur la peau, en particulier l'eau de la Roche Posay. De telles eaux thermales ont généralement une forte teneur en électrolytes, comprenant notamment des sels hydrosolubles de métaux alcalino-terreux. Bien entendu la quantité de métaux alcalino-terreux dans l'eau thermale sera prise en compte pour déterminer la quantité totale de métaux alcalino-terreux dans la composition selon l'invention.

Ladite phase aqueuse peut être présente à une teneur comprise entre 30 % et 80 % en poids par rapport au poids total de la composition, de préférence comprise entre 40 % et 70 % en poids.

La phase grasse de l'émulsion selon l'invention peut comprendre des corps gras usuellement utilisés dans le domaine d'application envisagé. Parmi ceux-ci, on peut citer les corps gras siliconés tels que les huiles de silicone, ainsi que les corps gras non siliconés tels que les huiles végétales, minérales, animales ou synthétiques.

Parmi les corps gras siliconés, on peut citer :

- (i) les polyalkyl($C_1$-$C_{20}$) siloxanes et notamment ceux à groupements terminaux triméthylsilyle, parmi lesquels on peut citer les polydiméthylsiloxanes linéaires (PDMS) et les alkylméthylpolysiloxanes tels que la cétyldiméthicone (nom CTFA),
- (ii) les huiles siliconées volatiles, telles que :

  - les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
  - les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane,

Parmi les corps gras non siliconés, on peut citer les huiles usuelles, telles que l'huile de paraffine, de vaseline, l'huile d'amande, le perhydrosqualène, l'huile d'abricot, l'huile de germes de blé, d'amande douce, de callophyllum, de palme, de ricin, d'avocat, de jojoba, d'olive ou de germes de céréales; des esters d'acides gras ou d'alcools gras, telles que l'octyl dodécyl myristate ou les benzoates d'alkyle en $C_{12}$-$C_{15}$, des alcools; des acétylglycérides; des octanoates, décanoates ou ricinoléates d'alcools ou de polyalcools; le polyisobutène hydrogéné, des huiles hydrogénées concrètes à 25°C; des lanolines; des esters gras concrets à 25°C.

Ces corps gras peuvent en particulier être choisis de manière variée par l'homme du métier afin de préparer une composition ayant les propriétés souhaitées, par exemple en consistance ou en texture.

Ainsi, la phase grasse de l'émulsion selon l'invention peut être présente à une teneur comprise entre 20 % et 70 % en poids par rapport au poids total de la composition et de préférence comprise entre 20 % et 50 % en poids.

En outre, la composition selon l'invention peut comprendre au moins un agent stabilisant approprié pour une émulsion H/E à forte teneur en électrolyte. De tels stabilisants sont choisis notamment parmi les alcools gras ou les acides gras en $C_6$ à $C_{20}$, les gélifiants hydrosolubles comme les gommes de xanthane, de caroube, de guar, de carraghénanes ou de géllane.

La quantité d'agent stabilisant est avantageusement comprise entre 0 et 10 % en poids par rapport au poids total de la composition, de préférence comprise entre 0,1 et 5 % en poids.

Les compositions selon l'invention se présentent avantageusement sous forme de crème, de lait, etc.

De façon connue, l'émulsion de l'invention peut contenir également des adjuvants habituels dans les domaines cosmétique et/ou dermatologique, tels que les conservateurs, les antioxydants, les agents complexants, les solvants, les parfums, les charges (par exemple un agent matifiant), les filtres, les bactéricides, les absorbeurs d'odeur, les matières colorantes, etc. Les quantités de ces différents adjuvants sont celles classiquement utilisées dans le domaine considéré, et par exemple de 0,01 à 5 % en poids du poids total de la composition. Ces adjuvants, selon leur nature, peuvent être introduits dans la phase grasse ou dans la phase aqueuse.

La composition selon l'invention peut également comprendre au moins un composé actif susceptible de provoquer une irritation cutanée.

La composition selon l'invention peut également être employée pour diminuer l'effet irritant d'au moins un composé actif administré séparément dans une composition cosmétique ou pharmaceutique, par voie topique (crème, lotion, gel, etc.) ou systémique (orale, rectale, parentérale, etc). La composition selon l'invention peut être appliquée sur la peau simultanément à l'administration de l'actif susceptible d'irriter, ou de manière décalée dans le temps.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits bactériens ou végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

Ces actifs peuvent être destinés notamment à la prévention et/ou au traitement des affections cutanées. Parmi ces agents actifs, on peut citer à titre d'exemple :

- les agents modulant la différenciation et/ou la prolifération et/ou la pigmentation cutanée tels que l'acide rétinoïque et ses isomères, le rétinol et ses esters, les rétinoïdes notamment ceux décrits dans les demandes de brevet FR 2 570 377, EP 199 636, EP 325 540, EP 402 072, le rétinal, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antimétabolites ;
- les agents anti-chute des cheveux comme le minoxidil ;
- les antiseptiques.

La composition selon l'invention peut également comprendre comme actif au moins un agent kératolytique et/ou au moins un antagoniste de neuropeptide et/ou au moins un antagoniste de médiateur de l'inflammation, différents des sels métalliques hydrosolubles, notamment pour traiter les peaux sensibles.

Comme agents kératolytiques utiles selon l'invention, on peut citer que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique. De manière avantageuse, ces agents kératolytiques peuvent être présents dans la composition selon l'invention en des quantités allant jusqu'à 10 % en poids par rapport au poids total de la composition, de préférence compris entre 0,1 et 5 % en poids.

Comme antagonistes de neuropeptides utiles dans l'invention, on peut citer les antagonistes de substance P et les antagonistes de CGRP et comme antagonistes de médiateurs de l'inflammation, on peut citer les antagonistes d'histamine, d'interleukine 1 ou de TNFa. Ces antagonistes peuvent être présents à raison de 0,000001 à 10 % en poids du poids total de la composition et de préférence de 0,0001 à 5 %.

De façon avantageuse, on utilise de préférence des antagonistes réceptoriels de substance P, de CGRP et/ou d'interleukine 1.

Comme antagoniste de substance P utiles selon l'invention, on peut citer toute substance d'origine organique ou minérale, capable de produire une inhibition de la fixation réceptorielle de substance P ou une inhibition de la synthèse et/ou la libération de substance P par des fibres nerveuses sensitives.

L'antagoniste réceptoriel de substance P peut être notamment un peptide ou un dérivé non peptidique comportant un hétéroatome, et plus précisément un composé comportant un hétérocycle ou un hétéroatome lié directement ou indirectement à un cycle benzènique.

On peut utiliser par exemple comme peptide antagoniste de substance P réceptoriel le sendide et le spantide II.

On peut également utiliser dans l'invention comme peptide ceux décrits dans les brevets et demandes de brevet US 4 472 305, US 4 839 465, EP 101 929, EP 333 174, EP 336 230, EP 394 989, EP 443 32, EP 498 069, EP 515 681, EP 517 589, WO 92/22569 et GB 2 216 529.

Les antagonistes réceptoriels de substance P non peptidiques utiles selon l'invention sont notamment des composés hétérocycliques notamment soufrés, azotés ou oxygénés ou des composés comprenant un atome d'azote lié directement ou indirectement à un cycle benzénique.

Comme composé hétérocyclique, on peut utiliser selon l'invention ceux décrits dans les demandes de brevet suivantes : EP 360 390, EP 429 366, EP 430 771, EP 499 313, EP 514 273, EP 514 274, EP 514 275, EP 514 276, EP 520 555, EP 528 495, EP 532 456, EP 545 478, EP 558 156, WO 90/05525, WO 90/05729, WO 91/18878, WO 91/18899, WO 92/12151, WO 92/15585, WO 92/17449, WO 92/20676, WO 93/00330, WO 93/00331, WO 93/01159, WO 93/01169, WO 93/01170, WO 93/06099, WO 93/09116 et WO 94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme composés comportant un atome d'azote lié directement ou indirectement à un noyau benzénique, on peut citer ceux décrits dans les demandes de brevet EP 522 808 et WO 93/01165.

Comme antagoniste de CGRP utiles selon l'invention, on peut citer toute substance d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante de présenter une activité pharmacologique antagoniste du CGRP, c'est-à-dire induire une réponse pharmacologique cohérente notamment dans l'un des tests suivants:

- la substance antagoniste doit diminuer la vasodilation induite par la capsaïcine et/ou
- la substance antagoniste doit provoquer une inhibition de la libération de CGRP par les fibres nerveuses sensitives et/ou
- la substance antagoniste doit provoquer une inhibition de la contraction du muscle lisse du canal déférent induite par le CGRP.

En outre, l'antagoniste de CGRP peut avoir une affinité pour les récepteurs au CGRP.

On peut utiliser dans l'invention par exemple comme antagoniste réceptoriel de CGRP, le CGRP 8-37, un anticorps anti-CGRP.

Lorsque la composition selon l'invention comprend un composé actif susceptible de provoquer une irritation cutanée choisi parmi les agents kératolytiques, les antagonistes de neuropeptide et les antagonistes de médiateur de l'inflammation, différents des sels métalliques hydrosolubles, la quantité de système émulsionant sera appropriée pour obtenir une composition stable, selon les critères de stabilité définis ci-dessus. Là encore, on cherchera à employer la quantité minimum de système émulsionant permettant d'obtenir une composition stable. Une quantité appropriée de système émulsionant est donc une quantité nécessaire et suffisante pour obtenir une composition stable au moins 1 mois à 45°C. Elle est de préférence inférieure à 10 % en poids par rapport au poids total de la composition, plus préférentiellement inférieure à 8 % en poids.

Les exemples ci-après d'émulsions selon l'invention sont des exemples donnés à titre d'illustration et sans caractère limitatif. Les quantités y sont données en % en poids par rapport au poids total de la composition.

Exemple 1 : Crème H/E

| Ingrédients | % |
|---|---|
| A: Phase grasse: | |
| Mélange de stéarate de PEG-100 et de mono/distéarate de glycérol (particulier commercialisé sous la dénomination Arlacel 165 par la société ICI) | 1,2 |
| Stéarate de PEG-20 (commercialisés sous la dénomination Myrj 49 par la société ICI) | 1,2 |
| Acide stéarique | 0,6 |
| Alcool cétylique | 0,6 |
| Alcool stéarylique | 0,6 |
| Cyclométhicone | 7,0 |
| Ester gras | 19,0 |
| Huile végétale | 4,0 |
| B: Phase aqueuse: | |
| Glycérine | 3,0 |
| Conservateurs | qs |
| Chlorure de strontium, 6 $H_2O$ | 5,5 |
| Eau distillée | qsp 100 |
| C: Phase annexe: | |
| Gomme de xanthane | 0,2 |
| Eau distillée | 10,0 |

La crème H/E est préparée selon le mode opératoire suivant:

- on fait chauffer les phases A et B à 75°C séparément, puis on verse progressivement à 600 tr/mn la phase A dans la phase B;
- on laisse refroidir sous agitation à 600 tr/mn jusqu'à 40°C;
- on ajoute alors la phase C à 1500 tr/mn, puis on agite l'émulsion à 3000 tr/mn pendant 5 min.

On obtient une émulsion stable pendant au moins 1 mois à 45°C

Exemple 2 : Crème H/E

| Ingrédients | % |
|---|---|
| A: Phase grasse: | |
| Tristéarate de sorbitol (commercialisé sous la dénomination Span 65 par la société ICI) | 0,9 |
| Stéarate de PEG-40 (commercialisés sous la dénomination Myrj 52 par la société ICI) | 2,0 |
| Alcool cétylique | 4,0 |
| Stéarate de glycérol | 3,0 |
| Cyclométhicone | 10,0 |
| Isoparaffine hydrogénée (6-8 moles d'isobutylène) | 14,0 |
| B: Phase aqueuse: | |
| Conservateurs | qs |
| Chlorure de strontium, 6 $H_2O$ | 6,6 |
| Eau distillée | qsp 100 |

L'émulsion est préparée en suivant le mode opératoire de l'exemple 1 On obtient également une composition stable au moins 1 mois à 45°C.

**Revendications**

1. Composition sous forme d'émulsion H/E, caractérisée en ce qu'elle comprend au moins 2 % en poids par rapport au poids total de la composition d'au moins un sel métallique hydrosoluble, et de 2 à 4,5% en poids par rapport au poids total de la composition d'un système émulsionnant approprié, à l'exception des compositions comprenant 1 % en poids d'un gélifiant constitué par l'association d'un polyacrylamide, d'isoparaffine en $C_{13}$-$C_{14}$ et de laureth-7 (CTFA) (commercialisé sous la dénomination Sepigel 305 par la société SEPPIC).

2. Composition selon la revendication 1, caractérisée en ce qu'elle comprend au moins 3 % en poids d'au moins un sel métallique hydrosoluble, de préférence au moins 5 % en poids.

3. Composition selon l'une des revendications 1 ou 2, caractérisée en ce que les sels métalliques hydrosolubles sont choisis parmi les sels hydrosolubles de métaux alcalins, de métaux alcalino-terreux, de métaux de transition et de métaux des groupes 13 et 14 de la classification périodique des éléments.

4. Composition selon l'une des revendications 1 à 3, caractérisée en ce que les sels métalliques hydrosolubles sont choisis parmi les sels de lithium, de strontium, de baryum, d'yttrium, de néodyme, de gadolinium, de manganèse et de zinc, de préférence de strontium.

5. Composition selon l'une des revendications 1 à 4, caractérisée en ce que les sels métalliques hydrosolubles sont choisis parmi les carbonates, bicarbonates, sulfates, glycérophosphates, borates, chlorures, nitrates, acétates, hydroxydes, persulfates, les sels d'α-hydroxyacides, les sels d'acides aminés ou les sels d'acides gras.

6. Composition selon l'une des revendications 1 à 5, caractérisée en ce que la quantité de sels métalliques hydrosolubles est comprise entre 2 % et 20 % en poids par rapport au poids total de la composition, de préférence comprise entre 5 % et 10 % en poids.

7. Composition selon l'une des revendications 1 à 6, caractérisée en ce que le système émulsionnant approprié est constitué par au moins un ester d'acide gras et de polyéthylène glycol (PEG) et/ou au moins un ester d'acide gras et de polyol polyoxyéthyléné, associé éventuellement à au moins un ester d'acide gras et de polyol, appropriés pour la préparation d'émulsions H/E.

**8.** Composition selon la revendication 7, caractérisée en ce que le système émulsionant approprié est constitué par au moins un ester d'acide gras et de PEG et au moins un ester d'acide gras de polyol.

**9.** Composition selon l'une des revendications 7 ou 8, caractérisée en ce que les acides gras sont choisis parmi les acides aliphatiques linéaires ou ramifiés, saturés ou insaturés, comprenant au moins 10 atomes de carbone, de préférence entre 10 et 20 atomes de carbone, plus préférentiellement entre 12 et 18 atomes de carbone, plus particulièrement les acides laurique, palmitique, stéarique, isostéarique,et oléique.

**10.** Composition selon l'une des revendications 7 à 9, caractérisée en ce que l'ester d'acide gras et de PEG comprend n unités de PEG, n étant un entier au moins égal à 8, de préférence compris entre 10 et 100, plus préférentiellement compris entre 20 et 100.

**11.** Composition selon l'une des revendications 7 à 10, caractérisée en ce que les esters d'acide gras et de polyol polyoxyéthylénés sont choisis parmi le tristéarate de sorbitane oxyéthyléné (20-OE), le trioléate de sorbitane oxyéthyléné (20-OE), le monolaurate de sorbitane oxyéthyléné (5-OE), le monostéarate de sorbitan oxyéthyléné (20-OE), le monopalmitate de sorbitane oxyéthyléné (20-OE) et le monooléate de sorbitane oxyéthyléné (20-OE).

**12.** Composition selon l'une des revendications 7 à 11, caractérisée en ce que l'ester d'acide gras et de polyol est choisi parmi les mono ou polyesters d'acides gras et de polyol, le polyol étant choisi parmi les alcools polyhydriques en $C_3$-$C_6$, comprenant 3 à 6 hydroxyles, en particulier les sucres et le glycérol.

**13.** Composition selon la revendication 12, caractérisée en ce que les sucres sont choisis parmi les sucres d'origine naturelle ou synthétique en $C_5$-$C_6$, plus particulièrement le sorbitol.

**14.** Composition selon la revendication 13, caractérisée en ce que les esters d'acide gras et de sucre sont choisis parmi les mono- ou triesters d'acides gras et de sorbitol, en particulier le tristéarate de sorbitol.

**15.** Composition selon la revendication 12, caractérisée en ce que les esters d'acide gras et de glycérol, sont choisis parmi les mono- ou diesters d'acide gras et de glycérol, en particulier le mono- ou distéarate de glycérol, et leurs mélanges en toutes proportions.

**16.** Composition selon l'une des revendications 7 à 15, caractérisée en ce que le rapport pondéral ester d'acide gras et de PEG / ester d'acide gras et de polyol est compris entre 0,2 et 3, de préférence compris entre 0,25 et 2,5.

**17.** Composition selon l'une quelconque des revendications précédnetes, caractérisée en ce que le système émulsionnant présente une balance HLB supérieure ou égale à 9, avantageusement supérieure ou égale à 11.

**18.** Composition selon l'une des revendications 1 à 17, caractérisée en ce qu'elle comprend au moins un antagoniste de neuropeptide et/ou au moins un antagoniste de médiateur de l'inflammation, différents des sels métalliques hydrosolubles.

**19.** Composition selon l'une des revendications 1 à 18, caractérisée en ce qu'elle comprend en outre des composés actifs susceptibles de provoquer une irritation cutanée.

**20.** Produit de combinaison comprenant une composition selon l'une des revendications 1 à 18 et une composition cosmétique ou pharmaceutique comprenant au moins un composé actif susceptible de provoquer une irritation cutanée, pour une utilisation séparée, simultanée ou décalée dans le temps pour diminuer l'effet irritant du composé actif administré séparément.